# EUROPEAN PATENT APPLICATION

(11) **EP 4 342 371 A1**
(43) Date of publication of application: **27.03.2024**
(21) Application number: 22849722.8
(22) Date of filing: 21.06.2022
(51) Int. Cl.: A61B 5/0537, A61B 5/00

(54) **ELECTRONIC DEVICE FOR MEASURING BIOMETRICS, AND OPERATION METHOD THEREFOR**

(30) Priority: 29.07.2021 KR 20210099814
(71) Applicant: Samsung Electronics Co., Ltd., Suwon-si, Gyeonggi-do 16677 (KR)
(72) Inventor: LEE, Sehee, Suwon-si Gyeonggi-do 16677 (KR); JEON, Eunbee, Suwon-si Gyeonggi-do 16677 (KR)
(74) Representative: Appleyard Lees IP LLP
(86) International application number: PCT/KR2022/008735
(87) International publication number: WO 2023/008743

(57) **Abstract**

Various embodiments of the present disclosure may disclose an electronic device for measuring biometrics, and an operation method therefor. The disclosed wearable electronic device may comprise: first electrodes disposed on a first surface of the electronic device so as to be in contact with a first part of a user; second electrodes disposed on a second surface of the electronic device so as to be in contact with a second part of the user; and a processor which measures the body impedance of the user by using the first electrodes and the second electrodes, and which determines the degree of movement of the user during body impedance measurement on the basis of deviation in body impedance.

## Description

### Technical Field

The disclosure relates to an electronic device for measuring biometric information and an operation method thereof.

### Background Art

With the development of medicine and the extension of average life expectancy, interest in health care is increasing. In this regard, interest in medical devices is also increasing. The scope has expanded to not only various medical devices used in hospitals or inspection institutions, but also small and medium-sized medical devices installed in public institutions or small-sized medical devices and health care devices owned or carried by individuals.

A body composition analyzer, a type of health care device, measures body composition using bioelectrical impedance analysis (BIA), which is a method of analyzing body composition by precisely measuring the resistance of a human body according to the quantification of body composition such as water, protein, bone, and fat that make up a human body. BIA regards a human body as a combination of impedances, and thus allows current to flow through a human body and measures the voltage by the current, thereby measuring the impedance of the human body from the current and voltage.

### Disclosure of the Invention

### Technical Goals

With increasing interest in health care, biometric information is measured and managed through wearable electronic devices, but there are various difficulties in measuring biometric information because feedback on a measurement posture or state is not provided. For example, a user may have difficulties in intuitively knowing which electrode causes suspension of biometric information measurement as being not grounded during biometric information measurement, and may have the inconvenience of restarting biometric information measurement when the electrode is grounded again.

According to various embodiments disclosed in the present disclosure, it is possible to monitor various error situations that occur during biometric information measurement through a wearable electronic device and provide feedback suitable for a corresponding error situation to a user, thereby effectively resolving the error that has occurred and measuring biometric information with high accuracy.

### Technical Solutions

An electronic device that is wearable according to an embodiment may include first electrodes disposed on a first surface of the electronic device so as to be in contact with a first part of a user, second electrodes disposed on a second surface of the electronic device so as to be in contact with a second part of the user, and a processor configured to measure a body impedance of the user using the first electrodes and the second electrodes, and determine a degree of movement of the user during the body impedance measurement based on a deviation in the body impedance.

An electronic device that is wearable according to an embodiment may include first electrodes disposed on a first surface of the electronic device so as to be in contact with a first part of a user, second electrodes disposed on a second surface of the electronic device so as to be in contact with a second part of the user, and a processor configured to determine whether a first reference electrode having a contact impedance exceeding a first threshold impedance is present among the first electrodes, and provide the user with feedback inducing moisturization of the first part of the user in response to the first reference electrode having the contact impedance exceeding the first threshold impedance being present.

An operation method of an electronic device that is wearable according to an embodiment may include measuring a body impedance of a user using first electrodes and second electrodes, and determining a degree of movement of the user during the body impedance measurement based on a deviation in the body impedance, wherein the first electrodes may be disposed on a first surface of the electronic device so as to be in contact with a first part of the user, and the second electrodes may be disposed on a second surface of the electronic device so as to be in contact with a second part of the user.

### Effects

According to various embodiments, it is possible to monitor various error situations that occur during biometric information measurement through a wearable electronic device and provide feedback suitable for a corresponding error situation to a user, thereby effectively resolving the error that has occurred and measuring biometric information with high accuracy.

Further, according to various embodiments, a plurality of electrodes included in a wearable electronic device may be used to measure biometric information. When a 2 o'clock electrode and a 4 o'clock electrode on a side surface of an electronic device, among the plurality of electrodes, are not in contact with the body of a user, biometric information measurement may not start, and feedback regarding which electrode is not in contact with the body may be provided such that biometric information may be measured smoothly. In addition, when the user moves a lot during biometric information measurement, feedback for each step according to the degree of movement may be provided such that an error situation that has occurred may be effectively resolved.

In addition, various effects directly or indirectly ascertained through the present disclosure may be provided.

### Brief Description of Drawings

FIG. 1 is a block diagram of an electronic device in a network environment according to various embodiments.
FIGS. 2 and 3 are perspective views of an electronic device according to an embodiment.
FIG. 4 is an exploded perspective view of an electronic device according to an embodiment.
FIGS. 5 and 6 are views illustrating a process of measuring biometric information of a user according to an embodiment.
FIGS. 7 and 8 are diagrams illustrating feedback related to the grounding of first electrodes according to an embodiment.
FIGS. 9 and 10 are diagrams illustrating feedback related to the grounding of second electrodes according to an embodiment.
FIGS. 11 and 12 illustrate examples of feedback and guidance.
FIGS. 13 to 15 are diagrams illustrating feedback provided when a first part and a second part of a user come into contact with each other according to an embodiment.
FIGS. 16 and 17 are diagrams illustrating feedback related to a movement of a user during measurement according to an embodiment.
FIGS. 18 and 19 are diagrams illustrating feedback related to moisturization according to an embodiment.
FIG. 20 is a flowchart illustrating an operation method of an electronic device that is wearable according to an embodiment.
FIG. 21 is a diagram illustrating an electronic device that is wearable according to an embodiment.

### Best Mode for Carrying Out the Invention

Hereinafter, embodiments will be described in detail with reference to the accompanying drawings. When describing the embodiments with reference to the accompanying drawings, like reference numerals refer to like elements and any repeated description related thereto will be omitted.

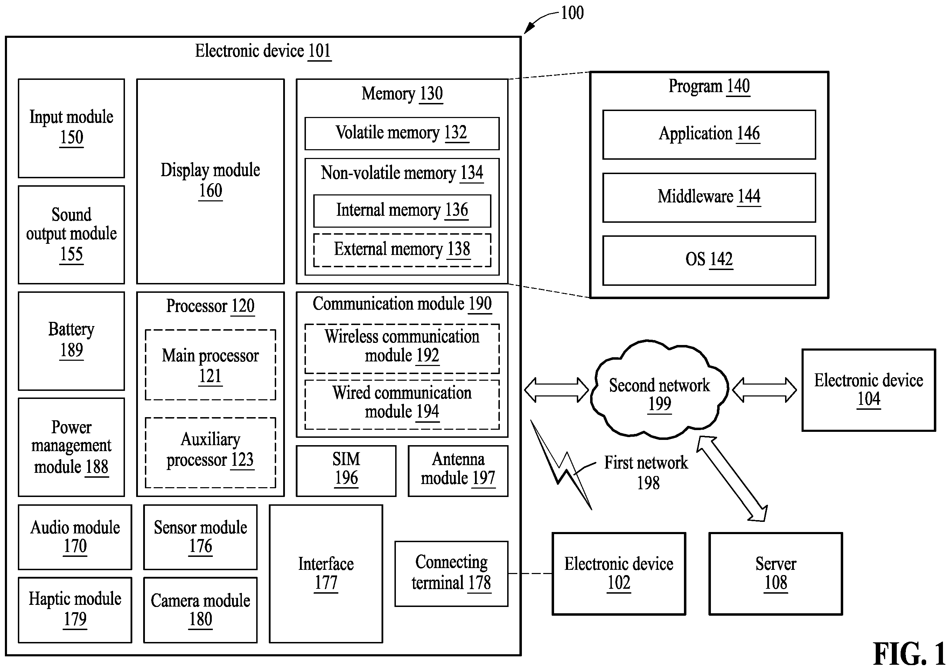

FIG. 1 is a block diagram illustrating an electronic device in a network environment according to an embodiment.

FIG. 1 is a block diagram illustrating an electronic device 101 in a network environment 100 according to various embodiments. Referring to FIG. 1, the electronic device 101 in the network environment 100 may communicate with an electronic device 102 via a first network 198 (e.g., a short-range wireless communication network), or communicate with at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, a memory 130, an input module 150, a sound output module 155, a display module 160, an audio module 170, and a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one (e.g., the connecting terminal 178) of the components may be omitted from the electronic device 101, or one or more other components may be added in the electronic device 101. In some embodiments, some of the components (e.g., the sensor module 176, the camera module 180, or the antenna module 197) may be integrated as a single component (e.g., the display module 160).

The processor 120 may execute, for example, software (e.g., a program 140) to control at least one other component (e.g., a hardware or software component) of the electronic device 101 connected to the processor 120, and may perform various data processing or computation. According to an embodiment, as at least a part of data processing or computation, the processor 120 may store a command or data received from another component (e.g., the sensor module 176 or the communication module 190) in a volatile memory 132, process the command or the data stored in the volatile memory 132, and store resulting data in a non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with the main processor 121. For example, when the electronic device 101 includes the main processor 121 and the auxiliary processor 123, the auxiliary processor 123 may be adapted to consume less power than the main processor 121 or to be specific to a specified function. The auxiliary processor 123 may be implemented separately from the main processor 121 or as a portion of the main processor 121.

The auxiliary processor 123 may control at least some of functions or states related to at least one (e.g., the display module 160, the sensor module 176, or the communication module 190) of the components of the electronic device 101, instead of the main processor 121 while the main processor 121 is in an inactive (e.g., sleep) state, or together with the main processor 121 while the main processor 121 is an active state (e.g., executing an application). According to an embodiment, the auxiliary processor 123 (e.g., an ISP or a CP) may be implemented as a portion of another component (e.g., the camera module 180 or the communication module 190) that is functionally related to the auxiliary processor 123. According to an embodiment, the auxiliary processor 123 (e.g., an NPU) may include a hardware structure specified for artificial intelligence model processing. An AI model may be generated through machine learning. Such learning may be performed, for example, by the electronic device 101 in which an artificial intelligence model is executed, or via a separate server (e.g., the server 108). Learning algorithms may include, but are not limited to, for example, supervised learning, unsupervised learning, semi-supervised learning, or reinforcement learning. The artificial intelligence model may include a plurality of artificial neural network layers. An artificial neural network may include, for example, a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network, or a combination of two or more thereof but is not limited thereto. The artificial intelligence model may, additionally or alternatively, include a software structure other than the hardware structure.

The memory 130 may store various pieces of data used by at least one component (e.g., the processor 120 or the sensor module 176) of the electronic device 101. The various pieces of data may include, for example, software (e.g., the program 140) and input data or output data for a command related thereto. The memory 130 may include the volatile memory 132 or the non-volatile memory 134.

The program 140 may be stored as software in the memory 130, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output a sound signal to the outside of the electronic device 101. The sound output module 155 may include, for example, a speaker or a receiver. The speaker may be used for general purposes, such as playing multimedia or playing record. The receiver may be used to receive an incoming call. According to an embodiment, the receiver may be implemented separately from the speaker or as a part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to sense a touch, or a pressure sensor adapted to measure the intensity of force incurred by the touch.

The audio module 170 may convert a sound into an electrical signal or vice versa. According to an embodiment, the audio module 170 may obtain the sound via the input module 150 or output the sound via the sound output module 155 or an external electronic device (e.g., the electronic device 102 such as a speaker or a headphone) directly or wirelessly connected to the electronic device 101.

The sensor module 176 may detect an operational state (e.g., power or temperature) of the electronic device 101 or an environmental state (e.g., a state of a user) external to the electronic device 101, and generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., by wire) or wirelessly. According to an embodiment, the interface 177 may include, for example, a high-definition multimedia interface (HDMI), a universal serial bus (USB) interface, a secure digital (SD) card interface, or an audio interface.

The connecting terminal 178 may include a connector via which the electronic device 101 may be physically connected to an external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electric signal into a mechanical stimulus (e.g., a vibration or a movement) or an electrical stimulus which may be recognized by a user via his or her tactile sensation or kinesthetic sensation. According to an embodiment, the haptic module 179 may include, for example, a motor, a piezoelectric element, or an electric stimulator.

The camera module 180 may capture a still image and moving images. According to an embodiment, the camera module 180 may include one or more lenses, image sensors, image signal processors, or flashes.

The power management module 188 may manage power supplied to the electronic device 101. According to an embodiment, the power management module 188 may be implemented as, for example, at least part of a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more CPs that are operable independently of the processor 120 (e.g., an AP) and that support a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module, or a power line communication (PLC) module). A corresponding one of these communication modules may communicate with the external electronic device 104 via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., a LAN or a wide area network (WAN))). These various types of communication modules may be implemented as a single component (e.g., a single chip), or may be implemented as multiple components (e.g., multiple chips) separate from each other. The wireless communication module 192 may identify and authenticate the electronic device 101 in a communication network, such as the first network 198 or the second network 199, using subscriber information (e.g., international mobile subscriber identity (IMSI)) stored in the SIM 196.

The wireless communication module 192 may support a 5G network after a 4G network, and a next-generation communication technology, e.g., a new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., a mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), an array antenna, analog beamforming, or a large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20 Gbps or more) for implementing eMBB, loss coverage (e.g., 164 dB or less) for implementing mMTC, or U-plane latency (e.g., 0.5 ms or less for each of downlink (DL) and uplink (UL), or a round trip of 1 ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element including a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in a communication network, such as the first network 198 or the second network 199, may be selected by, for example, the communication module 190 from the plurality of antennas. The signal or the power may be transmitted or received between the communication module 190 and the external electronic device via the at least one selected antenna. According to an embodiment, another component (e.g., a radio frequency integrated circuit (RFIC)) other than the radiating element may be additionally formed as a part of the antenna module 197.

According to various embodiments, the antenna module 197 may form a mmWave antenna module. According to an embodiment, the mmWave antenna module may include a PCB, an RFIC disposed on a first surface (e.g., the bottom surface) of the PCB or adj acent to the first surface and capable of supporting a designated high-frequency band (e.g., the mmWave band), and a plurality of antennas (e.g., array antennas) disposed on a second surface (e.g., the top or a side surface) of the PCB, or adjacent to the second surface and capable of transmitting or receiving signals in the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the external electronic devices 102 or 104 may be a device of the same type as or a different type from the electronic device 101. According to an embodiment, all or some of operations to be executed by the electronic device 101 may be executed at one or more external electronic devices (e.g., the external electronic devices 102 and 104, and the server 108). For example, if the electronic device 101 needs to perform a function or a service automatically, or in response to a request from a user or another device, the electronic device 101, instead of, or in addition to, executing the function or the service, may request one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an Internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

The electronic device according to various embodiments disclosed herein may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic device is not limited to those described above.

It should be appreciated that embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, "A or B", "at least one of A and B", "at least one of A or B", "A, B or C", "at least one of A, B and C", and "at least one of A, B, or C", each of which may include any one of the items listed together in the corresponding one of the phrases, or all possible combinations thereof. Terms such as "first", "second", or "first" or "second" may simply be used to distinguish the component from other components in question, and do not limit the components in other aspects (e.g., importance or order). It is to be understood that if an element (e.g., a first element) is referred to, with or without the term "operatively" or "communicatively", as "coupled with", "coupled to", "connected with", or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic", "logic block", "part", or "circuitry". A module may be a single integral component, or a minimum unit or part thereof, adapted to perform one or more functions. For example, according to an embodiment, the module may be implemented in a form of an application-specific integrated circuit (ASIC).

Embodiments as set forth herein may be implemented as software (e.g., the program 140) including one or more instructions that are stored in a storage medium (e.g., an internal memory 136 or an external memory 138) that is readable by a machine (e.g., the electronic device 101). For example, a processor (e.g., the processor 120) of the machine (e.g., the electronic device 101) may invoke at least one of the one or more instructions stored in the storage medium, and execute it. This allows the machine to be operated to perform at least one function according to the at least one instruction invoked. The one or more instructions may include code generated by a compiler or code executable by an interpreter. The machine-readable storage medium may be provided in the form of a non-transitory storage medium. Here, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments disclosed herein may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smartphones) directly. If distributed online, at least part of the computer program product may be temporarily generated or at least temporarily stored in the machine-readable storage medium, such as memory of the manufacturer's server, a server of the application store, or a relay server.

According to embodiments, each component (e.g., a module or a program) of the above-described components may include a single entity or multiple entities, and some of the multiple entities may be separately disposed in different components. According to an embodiment, one or more of the above-described components or operations may be omitted, or one or more other components or operations may be added. Alternatively or additionally, a plurality of components (e.g., modules or programs) may be integrated into a single component. In such a case, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

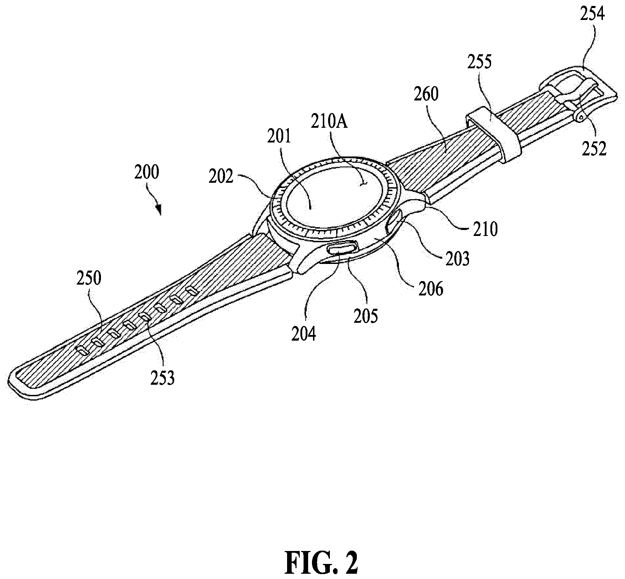

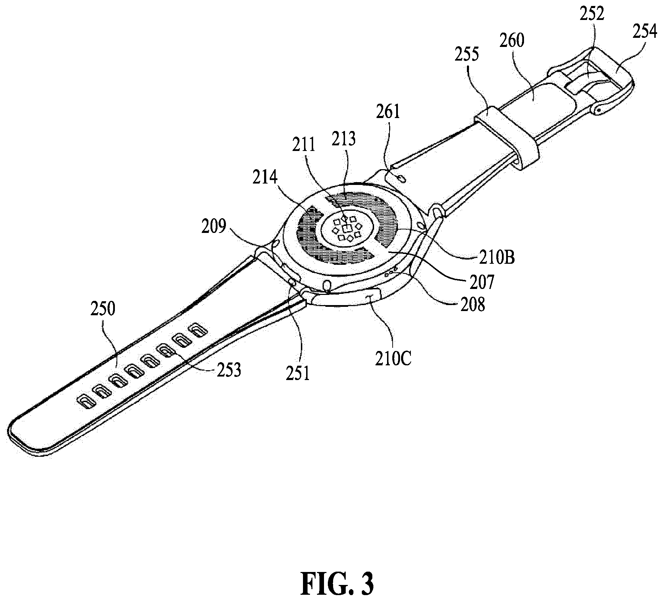

Referring to FIGS. 2 and 3, according to an embodiment, an electronic device 200 (e.g., the electronic device 101 of FIG. 1) may include a housing 210 including a first surface (or a front surface) 210A, a second surface (or a rear surface) 210B, and a side surface 210C surrounding a space between the first surface 210A and the second surface 210B, and fastening members 250 and 260 connected to at least a portion of the housing 210 and configured to detachably attach the electronic device 200 to a body part (e.g., a wrist, or an ankle) of a user. In another embodiment (not shown), the housing may also refer to a structure that forms a portion of the first surface 210A, the second surface 210B, and the side surface 210C of FIG. 2. According to an embodiment, the first surface 210A may be formed by a front plate 201 (e.g., a glass plate or a polymer plate including various coating layers) of which at least a portion is substantially transparent. The second surface 210B may be formed by a rear plate 207 that is substantially opaque. The rear plate 207 may be formed of, for example, coated or colored glass, ceramic, polymer, metal (e.g., aluminum, stainless steel (SS), or magnesium), or a combination of at least two thereof. The side surface 210C may be coupled to the front plate 201 and the rear plate 207 and may be formed by a side bezel structure (or a "side member") 206 including a metal and/or a polymer. In an embodiment, the rear plate 207 and the side bezel structure 206 may be integrally formed and may include the same material (e.g., a metal material such as aluminum). The fastening members 250 and 260 may be formed of various materials and may have various shapes. For example, the fastening members 250 and 260 may be formed of woven fabric, leather, rubber, urethane, metal, ceramic, or a combination of at least two of the aforementioned materials and may be implemented in an integrated form or with a plurality of unit links that are movable relative to each other.

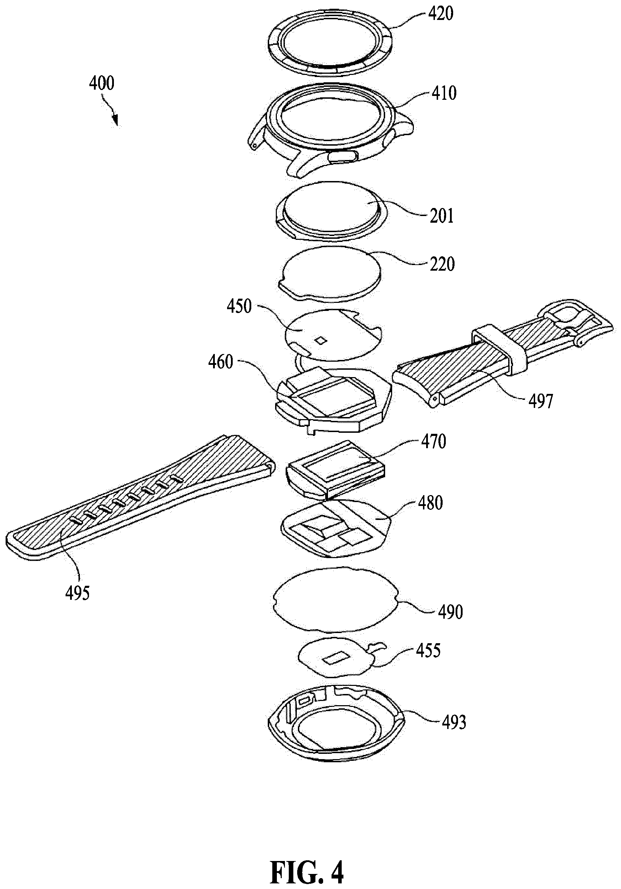

According to an embodiment, the electronic device 200 may include at least one of a display 220 (refer to FIG. 4), audio modules 205 and 208, a sensor module 211, key input devices 202, 203, and 204, and a connector hole 209. In an embodiment, the electronic device 200 may not include at least one (e.g., the key input devices 202, 203, and 204, the connector hole 209, or the sensor module 211) of the components, or additionally include other components.

The display 220 may be exposed through, for example, some portions of the front plate 201. The display 220 may have a shape corresponding to the shape of the front plate 201, and may have various shapes such as a circle, an oval, or a polygon. The display 220 may be coupled to or disposed adjacent to a touch sensing circuit, a pressure sensor capable of measuring an intensity (or pressure) of a touch, and/or a fingerprint sensor.

The audio modules 205 and 208 may include a microphone hole 205 and a speaker hole 208. A microphone for acquiring an external sound may be disposed in the microphone hole 205. In some embodiments, a plurality of microphones may be disposed to detect a direction of a sound. The speaker hole 208 may be used as an external speaker and a call receiver for calls. In an embodiment, the speaker hole 208 and the microphone hole 205 may be implemented as a single hole, or a speaker (e.g., a piezo speaker) may be included without the speaker hole 208.

The sensor module 211 may generate an electrical signal or a data value corresponding to an internal operating state of the electronic device 200 or an external environmental state. The sensor module 211 may include, for example, a biometric sensor module 211 (e.g., a heart rate monitor (HRM) sensor) disposed on the second surface 210B of the housing 210. The electronic device 200 may further include at least one of sensor modules (not shown), for example, a gesture sensor, a gyro sensor, an atmospheric pressure sensor, a magnetic sensor, an acceleration sensor, a grip sensor, a color sensor, an infrared (IR) sensor, a biometric sensor, a temperature sensor, a humidity sensor, or an illuminance sensor.

The sensor module 211 may include electrode areas 213 and 214 that form a portion of the surface of the electronic device 200 and a biosignal detection circuit (not shown) electrically connected to the electrode areas 213 and 214. For example, the electrode areas 213 and 214 may include a first electrode area 213 and a second electrode area 214 disposed on the second surface 210B of the housing 210. The sensor module 211 may be configured such that the electrode areas 213 and 214 obtain an electrical signal from a body part of the user and the biosignal detection circuit detects biometric information of the user based on the electrical signal.

The key input devices 202, 203, and 204 may include a wheel key 202 disposed on the first surface 210A of the housing 210 and rotatable in at least one direction, and/or side key buttons 203 and 204 disposed on the side surface 210C of the housing 210. The wheel key 202 may have a shape corresponding to the shape of the front plate 201. According to an embodiment, the key buttons 203 and 204 may include a conductive material and may be included in the sensor module 211 together with the electrode areas 213 and 214 and used to detect biometric information of the user. In an embodiment, the electronic device 200 may not include some or all of the above-described key input devices 202, 203, 204, and the key input devices 202, 203, and 204 that are not included may be implemented in other forms such as soft keys on the display 220.

The connector hole 209 may include another connector hole (not shown) that accommodates a connector (e.g., a universal serial bus (USB) connector) for transmitting and receiving power and/or data to and from an external electronic device and accommodates a connector for transmitting and receiving an audio signal to and from an external electronic device. The electronic device 200 may further include, for example, a connector cover (not shown) that covers at least a portion of the connector hole 209 and blocks infiltration of external foreign materials into the connector hole 209.

The fastening members 250 and 260 may be detachably fastened to at least a partial area of the housing 210 using locking members 251 and 261. The fastening members 250 and 260 may include one or more of a fixing member 252, a fixing member fastening hole 253, a band guide member 254, and a band fixing ring 255.

The fixing member 252 may be configured to fix the housing 210 and the fastening members 250 and 260 to a body part (e.g., a wrist, an ankle, etc.) of the user. The fixing member fastening hole 253 may correspond to the fixing member 252 to fix the housing 210 and the fastening members 250 and 260 to the body part of the user. The band guide member 254 may be configured to limit a range of a movement of the fixing member 252 when the fixing member 252 is fastened to the fixing member fastening hole 253, so that the fastening members 250 and 260 may be fastened to the body part of the user in a state of being brought into close contact with the body part of the user. The band fixing ring 255 may limit a range of a movement of the fastening member 250, 260 in a state in which the fixing member 252 and the fixing member fastening hole 253 are fastened with each other.

Referring to FIG. 4, an electronic device 400 (e.g., the electronic device 101 of FIG. 1 or the electronic device 200 of FIG. 2) may include a side bezel structure 410, a wheel key 420, a front plate 201, a display 220, a first antenna 450, a second antenna 455, a support member 460 (e.g., a bracket), a battery 470, a PCB 480, a sealing member 490, a rear plate 494, and fastening members 495 and 497. At least one of the components of the electronic device 400 may be the same as or similar to at least one of the components of FIG. 1 or the electronic device 200 of FIG. 2, and a repeated description thereof will be omitted hereinafter. The support member 460 may be disposed inside the electronic device 400 and connected to the side bezel structure 410, or may be integrally formed with the side bezel structure 410. The support member 460 may be formed of, for example, a metal material and/or a non-metal material (e.g., polymer). The display 220 may be connected to one surface of the support member 460, and the PCB 480 may be connected to another surface of the support member 460. The PCB 480 may be provided with a processor, a memory, and/or an interface mounted thereon. The processor may include, for example, one or more of a CPU, a GPU, an AP, a sensor processor, or a communication processor.

The memory may include, for example, a volatile memory or a non-volatile memory. The interface may include, for example, an HDMI, a USB interface, an SD card interface, or an audio interface. For example, the interface may electrically or physically connect the electronic device 400 to an external electronic device, and may include a USB connector, an SD card/multimedia card (MMC) connector, or an audio connector.

The battery 470, which is a device for supplying power to at least one component of the electronic device 400, may include, for example, a primary cell that is not rechargeable, a secondary cell that is rechargeable, or a fuel cell. For example, at least a portion of the battery 470 may be disposed on substantially the same plane as the PCB 480. The battery 470 may be disposed integrally inside the electronic device 200, or disposed detachably from the electronic device 200.

The first antenna 450 may be disposed between the display 220 and the support member 460. The first antenna 450 may include, for example, a near-field communication (NFC) antenna, a wireless charging antenna, and/or a magnetic secure transmission (MST) antenna. For example, the first antenna 450 may perform short-range communication with an external device, wirelessly transmit and receive power used for charging, or transmit a magnetism-based signal including a short-range communication signal or payment data. In another embodiment, an antenna structure may be formed by a portion of the side bezel structure 410 and/or the support member 460, or a combination thereof.

The second antenna 455 may be disposed between the PCB 480 and the rear plate 493. The second antenna 455 may include, for example, an NFC antenna, a wireless charging antenna, and/or an MST antenna. For example, the second antenna 455 may perform short-range communication with an external device, wirelessly transmit and receive power used for charging, or transmit a magnetism-based signal including a short-range communication signal or payment data. In another embodiment, an antenna structure may be formed by a portion of the side bezel structure 410 and/or the rear plate 493, or a combination thereof.

The sealing member 490 may be disposed between the side bezel structure 410 and the rear plate 494. The sealing member 490 may be configured to prevent moisture and foreign materials from being introduced into a space surrounded by the side bezel structure 410 and the rear plate 494 from the outside.

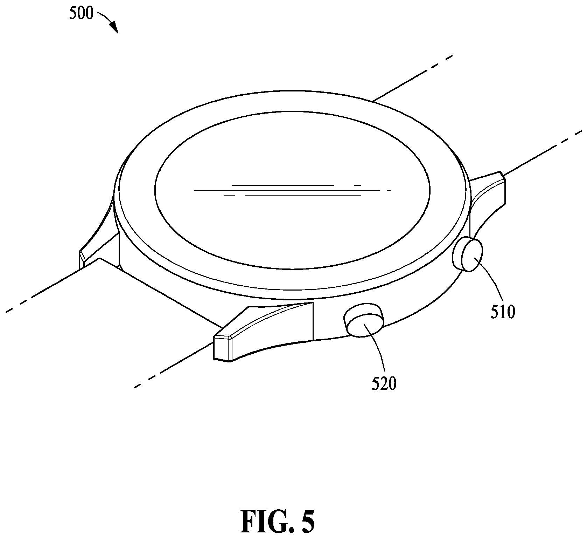

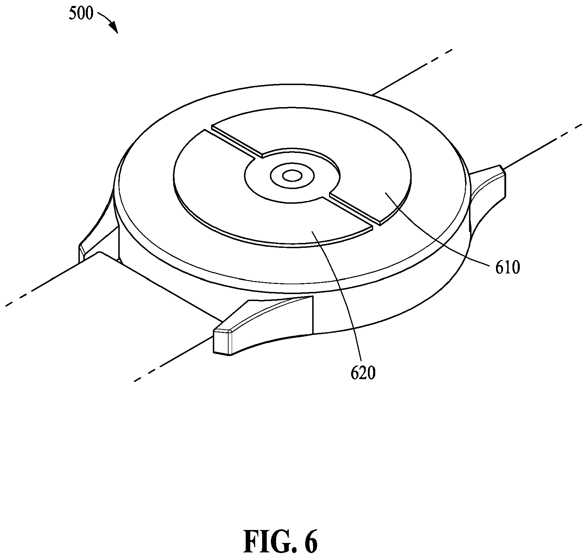

FIGS. 5 and 6 are views illustrating a process of measuring biometric information of a user according to an embodiment.

Referring to FIGS. 5 and 6, a wearable electronic device 500 (e.g., the electronic device 101 of FIG. 1, the electronic device 200 of FIG. 2, and the electronic device 400 of FIG. 4) may include electrodes 510 and 520 on a side surface and electrodes 610 and 620 on a rear surface. For ease of description, in this specification, the electrode 510 may be referred to as a 2 o'clock electrode, the electrode 520 may be referred to as a 4 o'clock electrode, the electrode 610 may be referred to as a 6 o'clock electrode, and the electrode 620 may be referred to as a 12 o'clock electrode. Also, in this specification, the electrodes 610 and 620 may be referred to as first electrodes, and the electrodes 510 and 520 may also be referred to as second electrodes.

The electronic device 500 may measure a body impedance using the first electrodes 610 and 620 and the second electrodes 510 and 520, and analyze body composition of a user using the body impedance. The body composition may include body fat, skin characteristics (e.g., body water), muscle strength, and the presence or absence of edema of a subject, but is not limited thereto.

When measuring a body impedance, the first electrodes 610 and 620 and the second electrodes 510 and 520 may come into contact with the body. The first electrodes 610 and 620 may naturally come into contact with the wrist of the user as the electronic device 500 is worn on the wrist of the user. The second electrodes 510 and 520 may be in contact with fingers of an arm not with the electronic device 500 on. For example, when the electronic device 500 is worn on the left wrist of the user, the second electrodes 510 and 520 may be in contact with the middle and ring fingers of the right hand, respectively. The first electrodes 610 and 620 and the second electrodes 510 and 520 may be electrically isolated.

When all the first electrodes 610 and 620 and the second electrodes 510 and 520 are in contact with the corresponding body parts, a closed loop may be formed by the left arm, torso, and right arm of the user, such that the body impedance may be measured. For example, in a first cycle, the electronic device 500 may apply current to the closed loop using the 2 o'clock electrode 510 and the 12 o'clock electrode 620 and measure the voltage on the closed loop using the 4 o'clock electrode 520 and the 6 o'clock electrode 610. In a second cycle, the electronic device 500 may apply current to the closed loop using the 4 o'clock electrode 520 and the 6 o'clock electrode 610 and measure the voltage on the closed loop using the 2 o'clock electrode 510 and the 12 o'clock electrode 620. The electronic device 500 may measure the body impedance of the user using the applied currents and the measured voltages.

Also, the electronic device 500 may measure a contact impedance of each of the first electrodes 610 and 620 and the second electrodes 510 and 520. With the contact between the body and each of the electrodes 510, 520, 610, and 620, each electrode may have a contact impedance. The contact impedance may change depending on the condition of the surface of the body (e.g., skin condition). Also, the contact impedance may change according to the frequency of an electrical signal applied. The contact impedance of each electrode may be measured using a closed loop between the first electrodes 610 and 620 or a closed loop between the second electrodes 510 and 520, rather than using the closed loop described above. As described in detail later, the electrode impedance may be used to determine whether each electrode is grounded. In this specification, whether an electrode is grounded may also be expressed as whether the corresponding electrode is in contact with the body.

To measure the body impedance of the user with high accuracy for accurate body composition measurement, a correct measurement posture may need to be maintained. It is possible to achieve high accuracy of body composition measurement by determining whether the measurement posture of the user is accurate and, if not, providing feedback about any errors in the posture of the user. In this specification, biometric information may include one or more of the body composition, the body impedance, and the contact impedance.

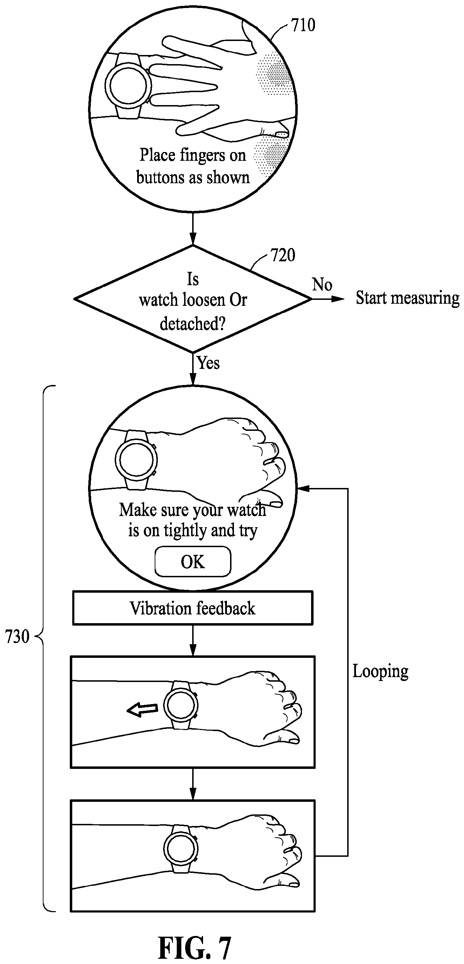

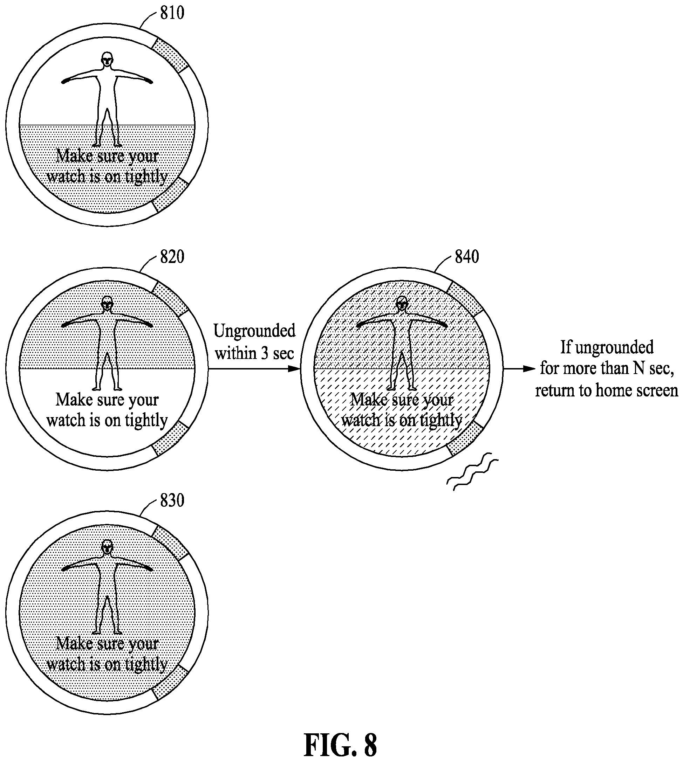

FIGS. 7 and 8 are diagrams illustrating feedback related to the grounding of first electrodes according to an embodiment.

Referring to FIG. 7, examples of determining whether first electrodes (e.g., the electrodes 610 and 620 of FIG. 6) are grounded, by an electronic device (e.g., the electronic device 101 of FIG. 1, the electronic device 200 of FIG. 2, the electronic device 400 of FIG. 4, and the electronic device 500 of FIG. 5), are shown. In this specification, the grounding of an electrode may indicate that the corresponding electrode is in contact with the body (e.g., the wrist or fingers) of the user.

In operation 710, the electronic device may display a screen guiding the start of biometric information measurement.

In operation 720, the electronic device may determine whether the electronic device is worn on the wrist of the user. For example, the electronic device may determine whether the first electrodes disposed on the rear surface are in contact with the wrist of the user, worn loosely, or detached. The electronic device may measure a contact impedance of each of the first electrodes and determine whether the contact impedance of each of the first electrodes exceeds a third threshold impedance. Here, the third threshold impedance may be 80,000 ohms, but is not limited thereto. If the contact impedance of at least one of the first electrodes exceeds the third threshold impedance, the electronic device may determine that at least one of the first electrodes is not in contact with the wrist of the user, and may subsequently perform operation 730. Conversely, if the contact impedance of each of the first electrodes is less than or equal to the third threshold impedance, the electronic device may determine that all of the first electrodes are in contact with the wrist of the user, and may start biometric information measurement.

In operation 730, the electronic device may provide the user with visual feedback inducing proper wearing of the electronic device and restart of biometric information measurement. For example, the visual feedback may include a picture and/or text that induces the user to wear the electronic device properly. The text may include "Make sure your watch is on tightly and try again", but is not limited thereto. In addition, the electronic device may provide the user with clear feedback by providing vibration feedback together. For ease of description, in this specification, the visual feedback may also be referred to as a visual cue, and the vibration feedback may be referred to as haptic feedback.

The electronic device may provide the user with visual feedback inducing adjustment of the wearing position of the electronic device. When the user wears the electronic device on a bone protruding from the wrist (e.g., the ulna head), the electronic device may not be in tight contact with the wrist due to the protruding bone, and thus, the first electrodes may not be in contact with the wrist. To solve or prevent this phenomenon, the electronic device may provide the user with visual feedback inducing adjustment of the position of the electronic device.

The visual feedback described above may be replaced with or provided together with another type of feedback (e.g., sound feedback and/or vibration feedback) according to embodiments.

Referring to FIG. 8, examples of feedback provided by the electronic device (e.g., the electronic device 101 of FIG. 1, the electronic device 200 of FIG. 2, the electronic device 400 of FIG. 4, and the electronic device 500 of FIG. 5) when the first electrodes (e.g., the electrodes 610 and 620 of FIG. 6) are not grounded are shown.

In operation 810, the electronic device may provide the user with visual feedback through a first area of a display (e.g., the display module 160 and the display module 220 of FIG. 2 or FIG. 4), in a case where a 12 o'clock electrode of the first electrodes is not grounded. The first area of the display may be a portion corresponding to the 12 o'clock electrode, and may be, for example, a lower area of the display, but is not limited thereto. The visual feedback may include darkening (e.g., dimming) the first area, but is not limited thereto.

In operation 820, the electronic device may provide the user with visual feedback through a second area of the display, in a case where a 6 o'clock electrode of the first electrodes is not grounded. The second area of the display may be a portion corresponding to the 6 o'clock electrode, and may be, for example, an upper area of the display, but is not limited thereto. The visual feedback may include darkening the second area, but is not limited thereto.

In operation 830, the electronic device may provide the user with visual feedback through the first area and the second area of the display, in a case where the 6 o'clock electrode and the 12 o'clock electrode of the first electrodes are not grounded.

In operation 840, the electronic device may provide the user with visual feedback through the display, when the ungrounded 6 o'clock electrode is not grounded again and remains ungrounded for a determined time (e.g., 3 seconds). Since the biometric information measurement may be suspended if the ungrounded state is continuously maintained, the visual feedback may be provided through the first area as well as the second area of the display. The visual feedback may include screen flickering, but is not limited thereto. In addition, the electronic device may provide the user with more clear feedback by providing vibration feedback along with the visual feedback. The vibration feedback may be provided multiple times, and in this case, the intensity may also vary, but is not limited thereto.

If the ungrounded state is maintained for more than N seconds (where N is a positive number) after the visual feedback and the vibration feedback are provided, the electronic device may suspend the biometric information measurement and display a home screen on the display.

Although operation 840 shown as an example in FIG. 8 has been described based on the case where the 6 o'clock electrode of the first electrodes is not grounded (e.g., operation 820) for ease of description, the description may similarly apply to operation 810 or operation 830.

According to embodiments, a guide message may be provided together with the visual feedback described above in operations 810 to 840. Further, the visual feedback may be replaced with or provided together with another type of feedback (e.g., sound feedback and/or vibration feedback).

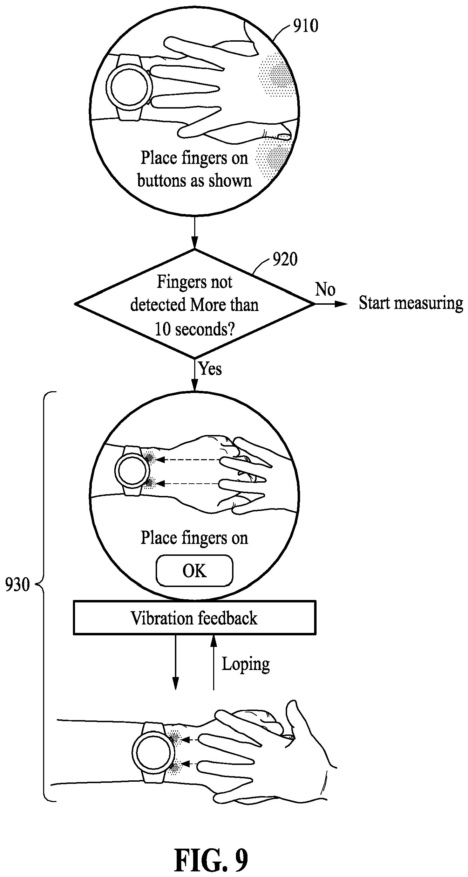

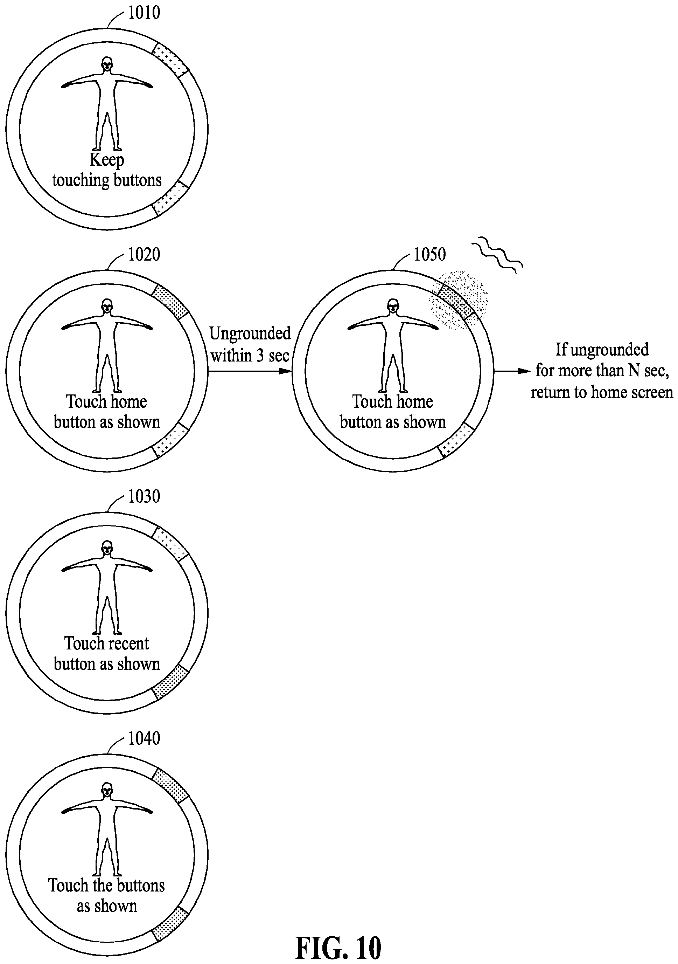

FIGS. 9 and 10 are diagrams illustrating feedback related to the grounding of second electrodes according to an embodiment.

Referring to FIG. 9, examples of determining whether second electrodes (e.g., the electrodes 510 and 520 of FIG. 5) are grounded, by an electronic device (e.g., the electronic device 101 of FIG. 1, the electronic device 200 of FIG. 2, the electronic device 400 of FIG. 4, and the electronic device 500 of FIG. 5), are shown.

In operation 910, the electronic device may display a screen guiding the start of biometric information measurement.

In operation 920, the electronic device may determine whether the fingers of a user are in contact with the second electrodes. For example, the electronic device may determine whether the fingers of the user are in contact with the second electrodes within a determined time (e.g., 10 seconds). The electronic device may measure a contact impedance of each of the second electrodes and determine whether the contact impedance of each of the second electrodes exceeds a third threshold impedance. Here, the third threshold impedance may be 80,000 ohms, but is not limited thereto. If the contact impedance of at least one of the second electrodes exceeds the third threshold impedance, the electronic device may determine that at least one of the second electrodes is not in contact with a finger of the user, and may subsequently perform operation 930. Conversely, if the contact impedance of each of the second electrodes is less than or equal to the third threshold impedance, the electronic device may determine that all of the second electrodes are in contact with the fingers of the user, and may start biometric information measurement.

In operation 930, the electronic device may provide the user with visual feedback inducing the touch of the second electrodes with the fingers. For example, the visual feedback may include a picture and/or text that induces the user to touch the second electrodes with the fingers. The text may include "Place your fingers on two buttons as shown and keep still during measurement", but is not limited thereto. In addition, the electronic device may provide the user with clear feedback by providing vibration feedback together.

The visual feedback described above may be replaced with or provided together with another type of feedback (e.g., sound feedback and/or vibration feedback) according to embodiments.

Referring to FIG. 10, examples of feedback provided by the electronic device (e.g., the electronic device 101 of FIG. 1, the electronic device 200 of FIG. 2, the electronic device 400 of FIG. 4, and the electronic device 500 of FIG. 5) according to whether the second electrodes (e.g., the electrodes 510 and 520 of FIG. 5) are grounded are shown. The electronic device may display feedback regarding whether each of the second electrodes is grounded on a partial area of a display (e.g., the display module 160 and the display 220 of FIG. 2 or FIG. 4) corresponding to the position of the corresponding electrode. For example, feedback regarding whether a 2 o'clock electrode of the second electrodes is grounded may be displayed at the 2 o'clock position on a circle displayed on the display of the electronic device, and feedback regarding whether a 4 o'clock electrode of the second electrodes is grounded may be displayed at a 4 o'clock position on the circle.

In operation 1010, the electronic device may display first visual feedback at the 2 o'clock position and the 4 o'clock position on the circle displayed on the display, in a case where all of the second electrodes are grounded.

In operation 1020, the electronic device may display second visual feedback at the 2 o'clock position and display the first visual feedback at the 4 o'clock position on the circle, in a case where the 2 o'clock electrode of the second electrodes is not grounded. The second visual feedback may be different from the first visual feedback, and may be feedback that draws the user's attention more than the first visual feedback, but is not limited thereto.

In operation 1030, the electronic device may display the first visual feedback at the 2 o'clock position and display the second visual feedback at the 4 o'clock position on the circle, in a case where the 4 o'clock electrode of the second electrodes is not grounded.

In operation 1040, the electronic device may display the second visual feedback at the 2 o'clock position and the 4 o'clock position on the circle, in a case where all of the second electrodes are not grounded.

In operation 1050, the electronic device may additionally provide the user with third visual feedback through the display, in a case where the ungrounded 2 o'clock electrode is not grounded again and remains ungrounded for a determined time (e.g., 3 seconds). Since the biometric information measurement may be suspended if the ungrounded state is continuously maintained, the third visual feedback may include screen flickering and/or feedback (e.g., edge lighting) with stronger brightness than the second visual feedback, but is not limited thereto. In addition, the electronic device may provide the user with more clear feedback by providing vibration feedback along with the visual feedback. The vibration feedback may be provided multiple times, and in this case, the intensity may also vary, but is not limited thereto.

If the ungrounded state is maintained for more than N seconds (where N is a positive number) after the visual feedback and the vibration feedback are provided, the electronic device may suspend the biometric information measurement and display a home screen on the display.

Although operation 1040 shown as an example in FIG. 10 has been described based on the case where the 2 o'clock electrode of the second electrodes is not grounded (e.g., operation 1020) for ease of description, the description may similarly apply to operation 1030 or operation 1040.

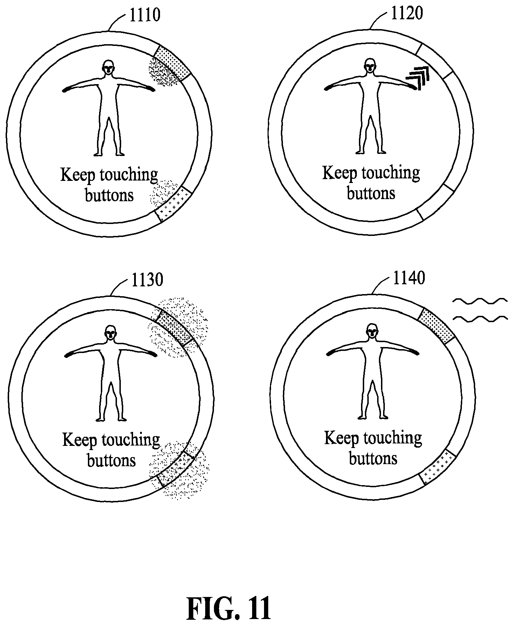

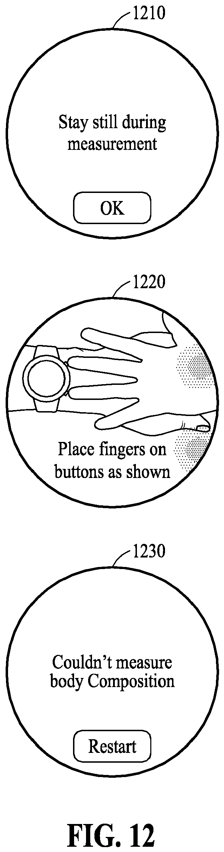

FIGS. 11 and 12 illustrate examples of feedback and guidance.

Referring to FIG. 11, various examples of feedback provided by an electronic device (e.g., the electronic device 101 of FIG. 1, the electronic device 200 of FIG. 2, the electronic device 400 of FIG. 4, and the electronic device 500 of FIG. 5) are shown.

In operation 1110, the electronic device may assign additional graphic elements to a partial area of a display (e.g., the display module 160 and the display 220 of FIG. 2 or FIG. 4) corresponding to the position of each of second electrodes (e.g., the electrodes 510 and 520 of FIG. 5). The graphic elements may include various visual effects, shapes, and colors without limitation.

In operation 1120, the electronic device may display an arrow pointing to an ungrounded electrode of the second electrodes. The arrow shown as an example in FIG. 11 may point to the ungrounded 2 o'clock electrode. However, the shape of the arrow is not limited thereto, and various shapes may apply without limitation.

In operation 1130, the electronic device may display edge lighting on a partial area of the display corresponding to the ungrounded electrode of the second electrodes.

In operation 1140, the electronic device may provide vibration feedback along with visual feedback. For example, the electronic device may provide vibration feedback to a portion corresponding to the ungrounded 2 o'clock electrode.

Referring to FIG. 12, various examples of guidance provided by an electronic device (e.g., the electronic device 101 of FIG. 1, the electronic device 200 of FIG. 2, the electronic device 400 of FIG. 4, and the electronic device 500 of FIG. 5) are shown.

In operation 1210, the electronic device may provide guidance on biometric information measurement in the form of text. The electronic device may provide an OK button together and receive whether everything is okay from the user. The guidance in the form of text and the OK button may be displayed in the form of a pop-up.

In operation 1220, the electronic device may provide guidance on biometric information measurement in the form of a picture as well as text. Through the guidance in the form of a picture, the user may receive intuitive guidance.

In operation 1230, the electronic device may provide a button interoperating with a subsequent action, in a case where feedback on an error occurring during biometric information measurement is provided to the user, but the error is not solved. For example, the electronic device may display a button indicating that biometric information measurement needs to be restarted due to an unresolved error.

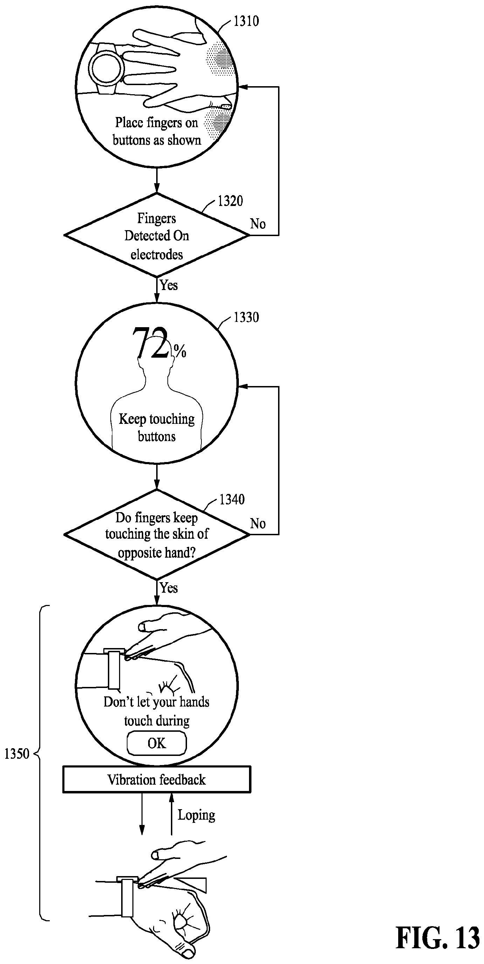

FIGS. 13 to 15 are diagrams illustrating feedback provided when a first part and a second part of a user come into contact with each other according to an embodiment.

Referring to FIG. 13, examples of determining whether both hands of a user are in contact, by an electronic device (e.g., the electronic device 101 of FIG. 1, the electronic device 200 of FIG. 2, the electronic device 400 of FIG. 4, and the electronic device 500 of FIG. 5) are shown.

In operation 1310, the electronic device may display a screen guiding the start of biometric information measurement.

In operation 1320, the electronic device may determine whether first electrodes (e.g., the electrodes 610 and 620 of FIG. 6) and second electrodes (e.g., the electrodes 510 and 520 of FIG. 5) are grounded. If at least one of the first electrodes and the second electrodes is not grounded, the electronic device may subsequently perform operation 1310. If all of the first electrodes and the second electrodes are grounded, the electronic device may subsequently perform operation 1330.

In operation 1330, the electronic device may measure biometric information. For example, the electronic device may measure a body impedance of the user and analyze body composition based on the measured body impedance. In this case, the electronic device may display a progress rate of impedance measurement or body composition analysis.

In operation 1340, the electronic device may determine whether the hands of the user are in contact with each other. The current applied during body impedance measurement may be alternating current and have a phase. Since the phase changes according to the length of the current flow, the length of the current flow may be calculated from the current phase value when the current output from the electronic device enters the electronic device again after flowing through the body.

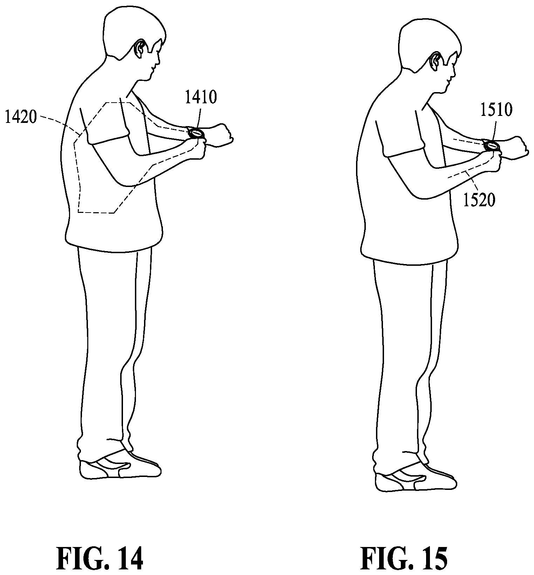

Referring to FIG. 14, in a case where biometric information is measured in a state in which the hands of the user are separated without touching each other, the current output from an electronic device 1410 (e.g., the electronic device 101 of FIG. 1, the electronic device 200 of FIG. 2, the electronic device 400 of FIG. 4, and the electronic device 500 of FIG. 5) may enter the electronic device 1410 again through a path 1420 going from the left arm to the torso and to the right arm.

On the other hand, referring to FIG. 15, in a case where biometric information is measured in a state in which the hands of the user are touching each other, the current output from an electronic device 1510 (e.g., the electronic device 101 of FIG. 1, the electronic device 200 of FIG. 2, the electronic device 400 of FIG. 4, the electronic device 500 of FIG. 5, and the electronic device 1410 of FIG. 14) may not pass through the torso but may enter the electronic device 1510 again through a path 1520 going from the left hand directly to the right hand.

Returning to FIG. 13, the electronic device may distinguish a difference between the current path length when the hands of the user are not in contact and the current path length when the hands of the user are in contact based on the current phase value. For example, the electronic device may determine that both hands are not in contact with each other if the phase value of the current that has passed through the body of the user and entered again is within a determined range. Conversely, the electronic device may determine that both hands are in contact with each other if the phase value of the current that has entered again is out of the determined range. For example, the determined range may include -4 degrees to 12 degrees or -1 degrees to 15 degrees, but is not limited thereto.

In operation 1340, in response to the determination that the hands of the user are not in contact with each other, the electronic device may sequentially perform operation 1330. Conversely, in response to the determination that the hands of the user are in contact with each other, the electronic device may sequentially perform operation 1350.

In operation 1350, the electronic device may provide the user with visual feedback inducing the user to keep both hands from touching each other. For example, the visual feedback may include a picture and/or text inducing separation of the hands from each other or indicating a state of the hands in contact with each other. The text may include "Don't let your hands touch during measurement", but is not limited thereto. In addition, the electronic device may provide the user with clear feedback by providing vibration feedback together.

The visual feedback described above may be replaced with or provided together with another type of feedback (e.g., sound feedback and/or vibration feedback) according to embodiments.

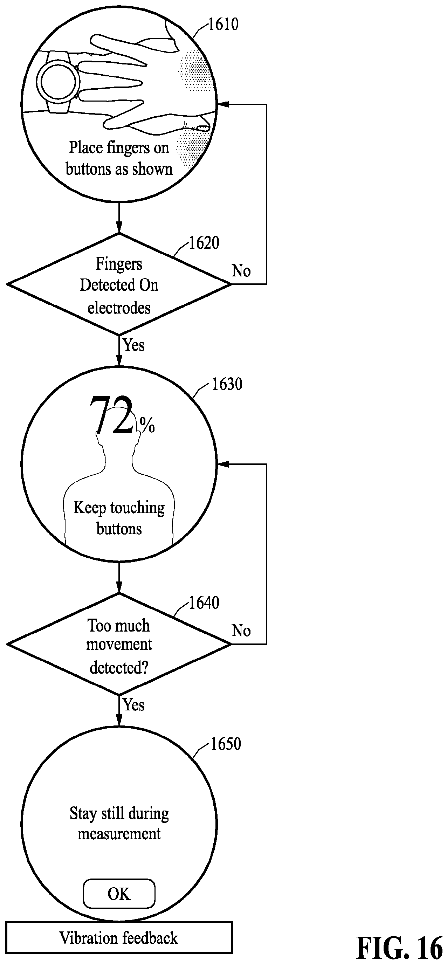

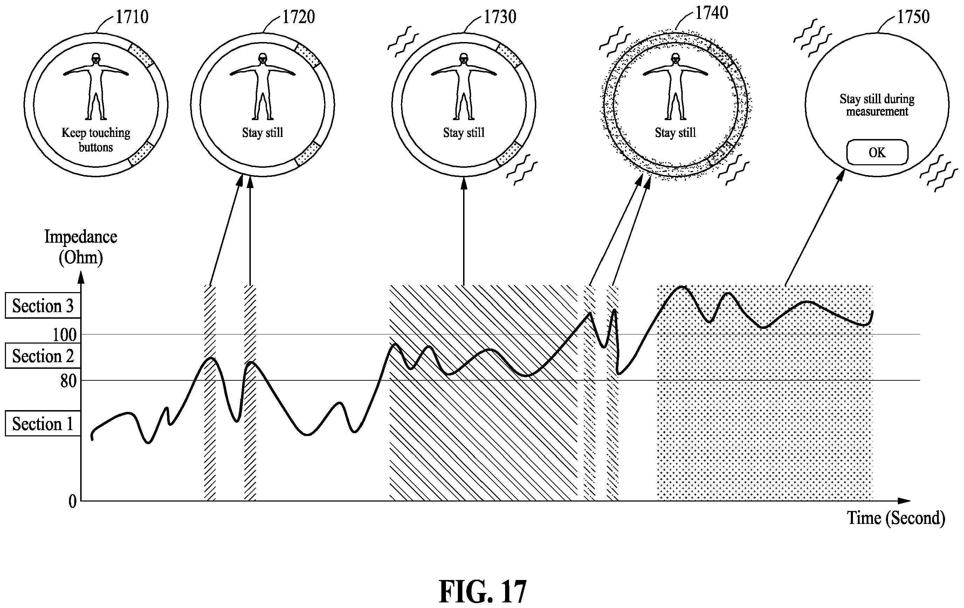

FIGS. 16 and 17 are diagrams illustrating feedback related to a movement of a user during measurement according to an embodiment.

Referring to FIG. 16, examples of determining a degree of movement of a user during biometric information measurement, by an electronic device (e.g., the electronic device 101 of FIG. 1, the electronic device 200 of FIG. 2, the electronic device 400 of FIG. 4, and the electronic device 500 of FIG. 5) are shown.

In operation 1610, the electronic device may display a screen guiding the start of biometric information measurement.

In operation 1620, the electronic device may determine whether first electrodes (e.g., the electrodes 610 and 620 of FIG. 6) and second electrodes (e.g., the electrodes 510 and 520 of FIG. 5) are grounded. If at least one of the first electrodes and the second electrodes is not grounded, the electronic device may subsequently perform operation 1610. If all of the first electrodes and the second electrodes are grounded, the electronic device may subsequently perform operation 1630.

In operation 1630, the electronic device may measure biometric information. For example, the electronic device may measure a body impedance of the user and analyze body composition based on the measured body impedance.

In operation 1640, the electronic device may determine whether the degree of movement of the user during biometric information measurement is within an allowable range. The electronic device may determine the degree of movement of the user based on a deviation in the body impedance of the user. For example, the electronic device may measure body impedance multiple times for a determined time period (e.g., 15 seconds), and determine a standard deviation of the measured body impedances to be the deviation in body impedance. The electronic device may determine that the movement of the user is considerable if the deviation in body impedance is large.

If the degree of movement of the user is within the allowable range, the electronic device may subsequently perform operation 1630. Conversely, if the degree of movement of the user is out of the allowable range, the electronic device may subsequently perform operation 1650.

In operation 1650, the electronic device may provide the user with visual feedback inducing a reduction of the movement of the user during biometric information measurement. For example, the visual feedback may include a picture and/or text inducing a reduction of movement. The text may include "Stay still during measurement", but is not limited thereto. In addition, the electronic device may provide the user with clear feedback by providing vibration feedback together. The visual feedback described above may be replaced with or provided together with another type of feedback (e.g., sound feedback and/or vibration feedback) according to embodiments.

The operation of determining whether the degree of movement of the user is within the allowable range and providing feedback accordingly will be described in detail with reference to FIG. 17.

Referring to FIG. 17, examples of feedback provided to a user according to a deviation in body impedance on a time axis, by an electronic device (e.g., the electronic device 101 of FIG. 1, the electronic device 200 of FIG. 2, the electronic device 400 of FIG. 4, and the electronic device 500 of FIG. 5) are shown. The electronic device may provide the user with feedback 1710 to 1750 for each step according to a deviation in body impedance.

In Section 1 where a deviation in body impedance does not exceed a first threshold value (e.g., 80 ohms) (e.g., no or very slight movement), the electronic device may provide the user with feedback 1710 inducing maintenance of a measurement posture while measuring biometric information of the user.

In a case where the deviation in body impedance enters Section 2 as exceeding the first threshold value, and then falls below the first threshold value again (e.g., intermittent occurrence of minute movement), for example, in a case where the deviation in body impedance exceeds the first threshold value for a first threshold time (e.g., 1 second), the electronic device may provide the user with feedback 1720 inducing reduction of the movement while maintaining the biometric information measurement for the user. The feedback 1720 may be visual feedback including text of "Stay still", but the text is not limited thereto. Here, the deviation in body impedance may be obtained based on a change in the body impedance value measured for a predetermined time period. The electronic device may continuously obtain a deviation in impedance based on a flow of an impedance value measured in a corresponding time period while shifting a time period of a predetermined length. In addition, the electronic device may verify an average impedance, a minimum impedance, or a maximum impedance along with the deviation in impedance in the corresponding time period. In addition, although the deviation in body impedance obtained by the electronic device on the time axis is shown as a graph in FIG. 17, the graph may be shown according to the magnitude of impedance instead of the deviation in impedance according to an embodiment. In this case, the description may be provided with the replacement of the deviation in impedance illustrated in FIG. 17 with the magnitude of impedance.

In a case where the deviation in body impedance exceeds the first threshold value for more than the first threshold time (e.g., continuous occurrence of minute movement), the electronic device may provide the user with feedback 1730 inducing reduction of the movement while maintaining the biometric information measurement for the user. The feedback 1730 may include visual feedback including text of "Stay still" and slight vibration feedback. However, the text is not limited thereto.

In a case where the deviation in body impedance enters Section 3 as exceeding a second threshold value (e.g., 100 ohms), and then falls below the second threshold value again (e.g., intermittent occurrence of significant movement), for example, in a case where the deviation in body impedance exceeds the second threshold value for a second threshold time (e.g., 1 second), the electronic device may provide the user with feedback 1740 inducing reduction of the movement while maintaining the biometric information measurement for the user. The feedback 1740 may include visual feedback including text of "Stay sill" and edge lighting, and strong vibration feedback. The vibration feedback provided as the feedback 1740 may be stronger than the feedback 1730. The text is not limited thereto. The second threshold time may be the same as the first threshold time, but may be shorter or longer than the first threshold time depending on embodiments.

In a case where the deviation in body impedance exceeds the second threshold value for more than the second threshold time (e.g., continuous occurrence of significant movement), the electronic device may suspend the biometric measurement for the user and provide feedback 1750. The feedback 1750 may include visual feedback in the form of a pop-up including text of "Stay still during measurement" and stronger vibration feedback. The vibration feedback provided as the feedback 1750 may be stronger than the feedback 1740. The text is not limited thereto.

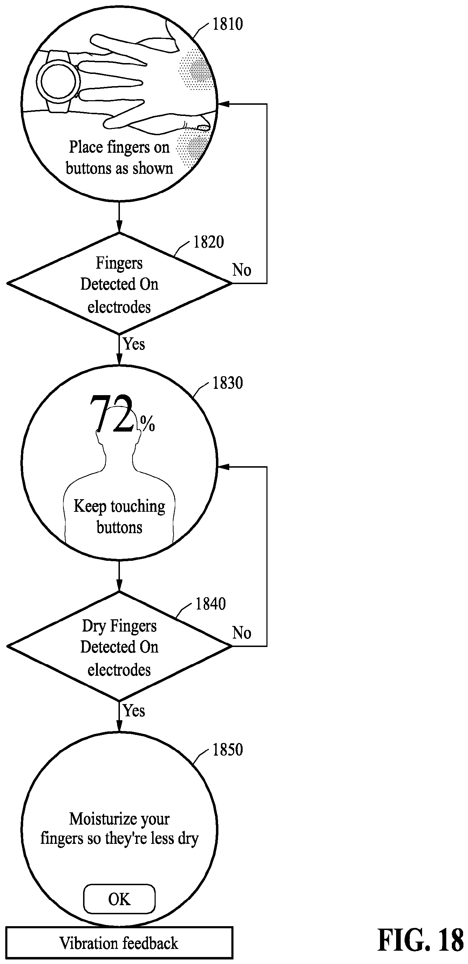

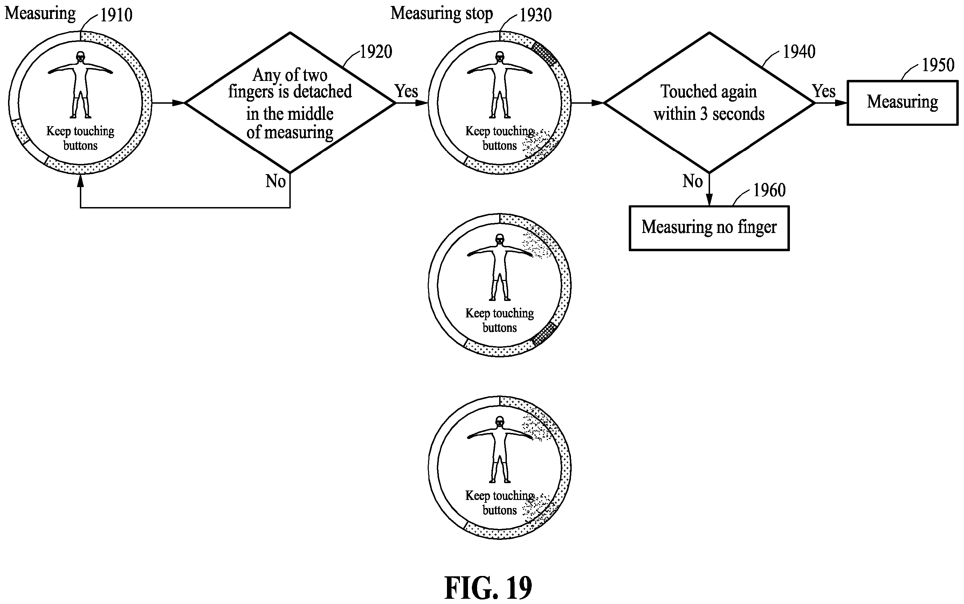

FIGS. 18 and 19 are diagrams illustrating feedback related to moisturization according to an embodiment.

Referring to FIG. 18, examples of determining a degree of movement of a user during biometric information measurement, by an electronic device (e.g., the electronic device 101 of FIG. 1, the electronic device 200 of FIG. 2, the electronic device 400 of FIG. 4, and the electronic device 500 of FIG. 5) are shown.

In operation 1810, the electronic device may display a screen guiding the start of biometric information measurement.

In operation 1820, the electronic device may determine whether first electrodes (e.g., the electrodes 610 and 620 of FIG. 6) and second electrodes (e.g., the electrodes 510 and 520 of FIG. 5) are grounded. If at least one of the first electrodes and the second electrodes is not grounded, the electronic device may subsequently perform operation 1810. If all of the first electrodes and the second electrodes are grounded, the electronic device may subsequently perform operation 1830. For example, if each of the first electrodes and the second electrodes is less than or equal to a third threshold impedance (e.g., 80,000 ohms), the electric device may determine that all of the first electrodes and the second electrodes are grounded.

In operation 1830, the electronic device may measure biometric information. For example, the electronic device may measure a body impedance of the user and analyze body composition based on the measured body impedance.

In operation 1840, the electronic device may determine whether a body part contacting each of the first electrodes and the second electrodes is dry. The electronic device may determine whether a body part contacting each electrode needs moisturization based on the contact impedance of each of the first electrodes and the second electrodes.

For example, the electronic device may measure a contact impedance of each of the first electrodes and determine whether the contact impedance of each of the first electrodes exceeds a first threshold impedance. Here, the first threshold impedance may be 10,000 ohms, but is not limited thereto. If the contact impedance of at least one of the first electrodes exceeds the first threshold impedance, the electronic device may determine that at least one of the first electrodes is dry and needs moisturization, and may subsequently perform operation 1850.

The electronic device may measure a contact impedance of each of the second electrodes and determine whether the contact impedance of each of the second electrodes exceeds a second threshold impedance. Here, the second threshold impedance may be 20,000 ohms, which is greater than the first threshold impedance, but is not limited thereto. If the contact impedance of at least one of the second electrodes exceeds the second threshold impedance, the electronic device may determine that at least one of the first electrodes is dry and needs moisturization, and may subsequently perform operation 1850.

If the contact impedance of each of the first electrodes is less than or equal to the first threshold impedance and the contact impedance of each of the second electrodes is less than or equal to the second threshold impedance, the electronic device may determine that the body part contacting each of the first electrodes and the second electrodes is not dry, and may subsequently perform operation 1830. In addition, although it is described above that the first threshold impedance and the second threshold impedance are different from each other, the first threshold impedance and the second impedance may be designated as the same single value (e.g., the first threshold impedance (10,000 ohms)). For example, if the contact impedances of all of the first electrodes and the second electrodes are less than 10,000 ohms, the electronic device may determine that the body parts in contact are not dry.

Referring to FIG. 19, an example of continuously performing biometric information measurement without suspension, if at least one electrode is ungrounded and then ground again within a determined time (e.g., 3 seconds) while an electronic device (e.g., the electronic device 101 of FIG. 1, the electronic device 200 of FIG. 2, the electronic device 400 of FIG. 4, and the electronic device 500 of FIG. 5) measures biometric information of a user, is shown.

In operation 1910, the electronic device may measure biometric information of the user using a plurality of electrodes.

In operation 1920, the electronic device may determine whether at least one of the plurality of electrodes is ungrounded during the biometric information measurement. For example, the electronic device may determine whether the contact impedance of each of the second electrodes (e.g., the electrodes 510 and 520 of FIG. 5) exceeds a third threshold impedance, thereby determining whether at least one of the second electrodes is ungrounded.

If the second electrodes continuously remain grounded, the electronic device may subsequently perform operation 1910. Conversely, if at least one of the second electrodes is ungrounded, the electronic device may subsequently perform operation 1930.

In operation 1930, if at least one of the second electrodes is ungrounded, the electronic device may suspend the biometric information measurement and display feedback on a partial area of a display (e.g., the display module 160 and the display 220 of FIG. 2 or FIG. 4) corresponding to the ungrounded electrode. For example, if a 4 o'clock electrode of the second electrodes is ungrounded, the electronic device may provide feedback at the 4 o'clock position on a circle displayed on the display. If a 2 o'clock electrode of the second electrodes is ungrounded, the electronic device may provide feedback at the 2 o'clock position on the circle displayed on the display. If all of the second electrodes are ungrounded, the electronic device may provide feedback at the 2 o'clock position and the 4 o'clock position on the circle displayed on the display.

In operation 1940, the electronic device may determine whether the body of the user touches the ungrounded electrode again within a determined time (e.g., 3 seconds). The electronic device may determine whether the contact impedance of the ungrounded electrode is less than or equal to the third threshold impedance. If the body of the user touches the ungrounded electrode again within the determined time, the electronic device may subsequently perform operation 1950. Conversely, if the body of the user does not touch the ungrounded electrode again within the determined time, the electronic device may subsequently perform operation 1960.

In operation 1950, the electronic device may restart the suspended biometric information measurement. The electronic device may restart the biometric information measurement from the time when the measurement is suspended, thereby preventing the inconvenience of having to perform the biometric information measurement again from the beginning and effectively improving the efficiency of biometric information measurement.

In operation 1960, the electronic device may terminate the biometric information measurement or perform biometric information measurement in a state in which at least one of the second electrodes is ungrounded.

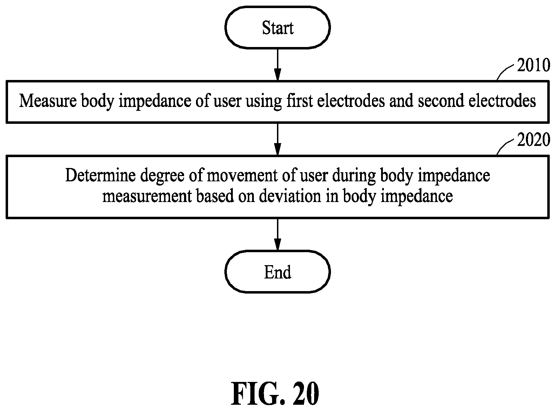

FIG. 20 is a flowchart illustrating an operation method of an electronic device that is wearable according to an embodiment.

In the following embodiments, operations may be performed sequentially, but are not necessarily performed sequentially. For example, the operations may be performed in different orders, and at least two of the operations may be performed in parallel. Operations 2010 and 2020 may be performed by an electronic device (e.g., the electronic device 101 of FIG. 1, the electronic device 200 of FIG. 2, the electronic device 400 of FIG. 4, and the electronic device 500 of FIG. 5).

In operation 2010, the electronic device may measure a body impedance of a user using first electrodes (e.g., the electrodes 610 and 620 of FIG. 6) and second electrodes (e.g., the electrodes 510 and 520 of FIG. 5). For example, the electronic device may measure the body impedance of the user multiple times, and determine a standard deviation of the measured body impedances to be a deviation in body impedance.

In operation 2020, the electronic device may determine a degree of movement of the user during the body impedance measurement based on the deviation in body impedance. For example, the electronic device may determine the degree of movement of the user based on a degree to which the deviation in body impedance exceeds a first threshold value.

The electronic device may provide the user with feedback determined according to the degree of movement. For example, the electronic device may provide the user with first visual feedback in a case where a time for which the deviation in body impedance exceeds the first threshold value is less than or equal to a first threshold time. In addition, the electronic device may provide the user with the first visual feedback and first vibration feedback in a case where the time for which the deviation in body impedance exceeds the first threshold value is greater than the threshold time.

The descriptions provided with reference to FIG. 1 to 19 may apply to the operations shown in FIG. 20, and thus a further detailed description thereof will be omitted.

According to an embodiment, an operation method of an electronic device that is wearable may include measuring a body impedance of a user using first electrodes and second electrodes, and determining a degree of movement of the user during the body impedance measurement based on a deviation in the body impedance, wherein the first electrodes may be disposed on a first surface of the electronic device so as to be in contact with a first part of the user, and the second electrodes may be disposed on a second surface of the electronic device so as to be in contact with a second part of the user.

According to an embodiment, the operation method of the electronic device may further include providing the user with feedback determined according to the degree of movement, wherein the determining of the degree of movement may include determining the degree of movement of the user based on a degree to which the deviation in body impedance exceeds a first threshold value.

According to an embodiment, in the operation method of the electronic device, the providing of the user with the feedback may include providing the user with first visual feedback in a case where a time for which the deviation in body impedance exceeds the first threshold value is less than or equal to a first threshold time, and providing the user with the first visual feedback and first vibration feedback in a case where the time for which the deviation in body impedance exceeds the first threshold value is greater than the threshold time.

According to an embodiment, in the operation method of the electronic device, the providing of the user with the feedback may include providing the user with second vibration feedback stronger than the first vibration feedback, the first visual feedback, and the second visual feedback in a case where a time for which the deviation in body impedance exceeds a second threshold value greater than the first threshold value is less than or equal to a second threshold time, and suspending measuring the body impedance and providing the user with third vibration feedback stronger than the second vibration feedback and third visual feedback in a case where the time for which the deviation in body impedance exceeds the second threshold value is greater than the second threshold time.

According to an embodiment, in the operation method of the electronic device, the measuring of the body impedance may include measuring the body impedance of the user multiple times, and determining a standard deviation of the measured body impedances to be the deviation in body impedance.

According to an embodiment, the operation method of the electronic device may further include determining whether a first reference electrode having a contact impedance exceeding a first threshold impedance is present among the first electrodes, and providing the user with feedback inducing moisturization of the first part of the user in response to the first reference electrode having the contact impedance exceeding the first threshold impedance being present, and/or determining whether a second reference electrode having a contact impedance exceeding a second threshold impedance is present among the second electrodes, and providing the user with feedback inducing moisturization of the second part of the user in response to the second reference electrode having the contact impedance exceeding the second threshold impedance being present.

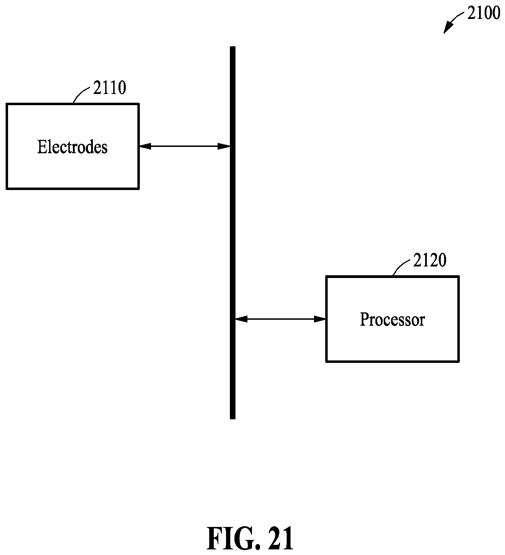

FIG. 21 is a diagram illustrating an electronic device that is wearable according to an embodiment.

Referring to FIG. 21, an electronic device 2100 (e.g., the electronic device 101 of FIG. 1, the electronic device 200 of FIG. 2, the electronic device 400 of FIG. 4, and the electronic device 500 of FIG. 5) according to an embodiment may include electrodes 2110 and a processor 2120 (e.g., the processor 120 of FIG. 1).

The electrodes 2110 may include first electrodes (e.g., the electrodes 610 and 620 of FIG. 6) disposed on a first surface (e.g., the second surface (or rear surface) 210B of FIG. 2) of the electronic device 2100 so as to be in contact with a first part (e.g., the wrist of any one of the left arm or right arm) of a user and second electrodes (e.g., the electrodes 510 and 520 of FIG. 5) disposed on a second surface (e.g., the side surface 210C of FIG. 2) of the electronic device so as to be in contact with a second part (e.g., fingers of the other one of the left arm or right arm) of the user.

The processor 2120 may perform the operations described above when instructions stored in a memory (e.g., the memory 130 of FIG. 1) are executed by the processor 2120. In addition, the electronic device 2100 may process the operations described above.

According to an embodiment, an electronic device 2100 that is wearable may include first electrodes disposed on a first surface of the electronic device 2100 so as to be in contact with a first part of a user, second electrodes disposed on a second surface of the electronic device 2100 so as to be in contact with a second part of the user, and a processor 2120 configured to measure a body impedance of the user using the first electrodes and the second electrodes, and determine a degree of movement of the user during the body impedance measurement based on a deviation in the body impedance.

According to an embodiment, the processor 2120 in the electronic device 2100 may be further configured to determine the degree of movement of the user based on a degree to which the deviation in body impedance exceeds a first threshold value, and provide the user with feedback determined according to the degree of movement.

According to an embodiment, the processor 2120 in the electronic device 2100 may be further configured to provide the user with first visual feedback in a case where a time for which the deviation in body impedance exceeds the first threshold value is less than or equal to a first threshold time, and provide the user with the first visual feedback and first vibration feedback in a case where the time for which the deviation in body impedance exceeds the first threshold value is greater than the threshold time.

According to an embodiment, the processor 2120 in the electronic device 2100 may be further configured to provide the user with second vibration feedback stronger than the first vibration feedback, the first visual feedback, and the second visual feedback in a case where a time for which the deviation in body impedance exceeds a second threshold value greater than the first threshold value is less than or equal to a second threshold time, and suspend measuring the body impedance and provide the user with third vibration feedback stronger than the second vibration feedback and third visual feedback in a case where the time for which the deviation in body impedance exceeds the second threshold value is greater than the second threshold time.

According to an embodiment, the processor 2120 in the electronic device 2100 may be further configured to measure the body impedance of the user multiple times, and determine a standard deviation of the measured body impedances to be the deviation in body impedance.

According to an embodiment, the processor 2120 in the electronic device 2100 may be further configured to determine whether a first reference electrode having a contact impedance exceeding a first threshold impedance is present among the first electrodes, and provide the user with feedback inducing moisturization of the first part of the user in response to the first reference electrode having the contact impedance exceeding the first threshold impedance being present, and determine whether a second reference electrode having a contact impedance exceeding a second threshold impedance is present among the second electrodes, and provide the user with feedback inducing moisturization of the second part of the user in response to the second reference electrode having the contact impedance exceeding the second threshold impedance being present, wherein the first threshold impedance may be less than the second threshold impedance.

According to an embodiment, the processor 2120 in the electronic device 2100 may be further configured to determine whether a third reference electrode having a contact impedance exceeding a third threshold impedance is present among the first electrodes and the second electrodes, and provide the user with feedback inducing the touch of the third reference electrode with a body of the user in response to the third reference electrode having the contact impedance exceeding the third threshold impedance being present.

According to an embodiment, the processor 2120 in the electronic device 2100 may be further configured to suspend measuring the body impedance when the third reference electrode having the contact impedance exceeding the third threshold impedance occurs while measuring the body impedance, and resume measuring the body impedance in a case where the contact impedance of the third reference electrode is less than or equal to the third threshold impedance within a threshold time after the suspension of measuring the body impedance.

According to an embodiment, the processor 2120 in the electronic device 2100 may be further configured to determine whether the first part and the second part are in contact with each other based on a phase value of a current flowing from any one of the first electrodes to any one of the second electrodes, and provide the user with feedback inducing separation of the first part and the second part from each other in response to the first part and the second part being in contact with each other.

According to an embodiment, the processor 2120 in the electronic device 2100 may be further configured to measure the body impedance by measuring a voltage from the other one of the first electrodes to the other one of the second electrodes when a current is applied from any one of the first electrodes to any one of the second electrodes and measuring a voltage from any one of the first electrodes to any one of the second electrodes when a current is applied from the other one of the first electrodes to the other one of the second electrodes.

According to an embodiment, an electronic device 2100 that is wearable may include first electrodes disposed on a first surface of the electronic device 2100 so as to be in contact with a first part of a user, second electrodes disposed on a second surface of the electronic device 2100 so as to be in contact with a second part of the user, and a processor 2120 configured to determine whether a first reference electrode having a contact impedance exceeding a first threshold impedance is present among the first electrodes, and provide the user with feedback inducing moisturization of the first part of the user in response to the first reference electrode having the contact impedance exceeding the first threshold impedance being present.

According to an embodiment, the processor 2120 in the electronic device 2100 that is wearable may be further configured to determine whether a second reference electrode having a contact impedance exceeding a second threshold impedance is present among the second electrodes, and provide the user with feedback inducing moisturization of the second part of the user in response to the second reference electrode having the contact impedance exceeding the second threshold impedance being present, wherein the first threshold impedance may be less than the second threshold impedance.

According to an embodiment, the processor 2120 in the electronic device 2100 that is wearable may be further configured to measure a body impedance of the user using the first electrodes and the second electrodes, and determine a degree of movement of the user during the body impedance measurement based on a deviation in the body impedance.

The embodiments of the present disclosure disclosed in the specification and the drawings are merely presented to easily describe technical contents of various embodiments of the present disclosure and help the understanding of them and are not intended to limit the various embodiments. Therefore, all changes or modifications derived from the technical idea of the various embodiments of the present disclosure as well as the embodiments disclosed herein should be construed to fall within the various embodiments.

## Claims

1. An electronic device that is wearable, comprising:
first electrodes disposed on a first surface of the electronic device so as to be in contact with a first part of a user;
second electrodes disposed on a second surface of the electronic device so as to be in contact with a second part of the user; and
a processor configured to measure a body impedance of the user using the first electrodes and the second electrodes, and determine a degree of movement of the user during the body impedance measurement based on a deviation in the body impedance.

2. The electronic device of claim 1, wherein
the processor is further configured to:
determine the degree of movement of the user based on a degree to which the deviation in body impedance exceeds a first threshold value, and provide the user with feedback determined according to the degree of movement.

3. The electronic device of claim 2, wherein
the processor is further configured to:
provide the user with first visual feedback in a case where a time for which the deviation in body impedance exceeds the first threshold value is less than or equal to a first threshold time, and
provide the user with the first visual feedback and first vibration feedback in a case where the time for which the deviation in body impedance exceeds the first threshold value is greater than the threshold time.

4. The electronic device of claim 3, wherein
the processor is further configured to:
provide the user with second vibration feedback stronger than the first vibration feedback, the first visual feedback, and the second visual feedback in a case where a time for which the deviation in body impedance exceeds a second threshold value greater than the first threshold value is less than or equal to a second threshold time, and
suspend measuring the body impedance and provide the user with third vibration feedback stronger than the second vibration feedback and third visual feedback in a case where the time for which the deviation in body impedance exceeds the second threshold value is greater than the second threshold time.

5. The electronic device of claim 1, wherein
the processor is further configured to:
measure the body impedance of the user multiple times, and determine a standard deviation of the measured body impedances to be the deviation in body impedance.

6. The electronic device of claim 1, wherein
the processor is further configured to:
determine whether a first reference electrode having a contact impedance exceeding a first threshold impedance is present among the first electrodes, and provide the user with feedback inducing moisturization of the first part of the user in response to the first reference electrode having the contact impedance exceeding the first threshold impedance being present, and
determine whether a second reference electrode having a contact impedance exceeding a second threshold impedance is present among the second electrodes, and provide the user with feedback inducing moisturization of the second part of the user in response to the second reference electrode having the contact impedance exceeding the second threshold impedance being present,
wherein the first threshold impedance is less than the second threshold impedance.

7. The electronic device of claim 1, wherein
the processor is further configured to:
determine whether a third reference electrode having a contact impedance exceeding a third threshold impedance is present among the first electrodes and the second electrodes, and provide the user with feedback inducing the touch of the third reference electrode with a body of the user in response to the third reference electrode having the contact impedance exceeding the third threshold impedance being present.

8. The electronic device of claim 7, wherein
the processor is further configured to:
suspend measuring the body impedance when the third reference electrode having the contact impedance exceeding the third threshold impedance occurs while measuring the body impedance, and
resume measuring the body impedance in a case where the contact impedance of the third reference electrode is less than or equal to the third threshold impedance within a threshold time after the suspension of measuring the body impedance.

9. The electronic device of claim 1, wherein
the processor is further configured to:
determine whether the first part and the second part are in contact with each other based on a phase value of a current flowing from any one of the first electrodes to any one of the second electrodes, and provide the user with feedback inducing separation of the first part and the second part from each other in response to the first part and the second part being in contact with each other.

10. The electronic device of claim 1, wherein
the processor is further configured to:
measure the body impedance by measuring a voltage from the other one of the first electrodes to the other one of the second electrodes when a current is applied from any one of the first electrodes to any one of the second electrodes and measuring a voltage from any one of the first electrodes to any one of the second electrodes when a current is applied from the other one of the first electrodes to the other one of the second electrodes.

11. An electronic device that is wearable, comprising:
first electrodes disposed on a first surface of the electronic device so as to be in contact with a first part of a user;
second electrodes disposed on a second surface of the electronic device so as to be in contact with a second part of the user; and
a processor configured to determine whether a first reference electrode having a contact impedance exceeding a first threshold impedance is present among the first electrodes, and provide the user with feedback inducing moisturization of the first part of the user in response to the first reference electrode having the contact impedance exceeding the first threshold impedance being present.

12. The electronic device of claim 11, wherein
the processor is further configured to:
determine whether a second reference electrode having a contact impedance exceeding a second threshold impedance is present among the second electrodes, and provide the user with feedback inducing moisturization of the second part of the user in response to the second reference electrode having the contact impedance exceeding the second threshold impedance being present,
wherein the first threshold impedance is less than the second threshold impedance.

13. The electronic device of claim 11, wherein
the processor is further configured to:
measure a body impedance of the user using the first electrodes and the second electrodes, and determine a degree of movement of the user during the body impedance measurement based on a deviation in the body impedance.

14. An operation method of an electronic device that is wearable, the operation method comprising:
measuring a body impedance of a user using first electrodes and second electrodes; and
determining a degree of movement of the user during the body impedance measurement based on a deviation in the body impedance,
wherein the first electrodes are disposed on a first surface of the electronic device so as to be in contact with a first part of the user, and
the second electrodes are disposed on a second surface of the electronic device so as to be in contact with a second part of the user.

15. The operation method of claim 14, further comprising:
providing the user with feedback determined according to the degree of movement,
wherein the determining of the degree of movement comprises:
determining the degree of movement of the user based on a degree to which the deviation in body impedance exceeds a first threshold value.
